# EUROPEAN PATENT APPLICATION

(11) **EP 2 127 656 A1**
(43) Date of publication of application: **02.12.2009**
(21) Application number: 06843916.5
(22) Date of filing: 20.12.2006
(51) Int. Cl.: A61K 31/662, A61K 31/593, A61P 19/10

(54) **COMPOSITIONS OF RISEDRONATE AND VITAMIN D3**

(71) Applicant: Landsteiner Scientific, S.A. De C.V., C.P. 01900, México D.F. (MX)
(72) Inventor: ARANDA FUENTES, Joaquín, C.P. 55266 Ecatepec (MX); HERNÁNDEZ PALMA, Elizabeth, C.P. 50200 Toluca (MX)
(74) Representative: Lauer, Joachim
(86) International application number: PCT/MX2006/000151
(87) International publication number: WO 2008/075930

(57) **Abstract**

The present invention is focused on compositions that contain risedronate and vitamin D as active ingredients. This dose combines the pharmaceutical properties of risedronate, which avoids bone degradation, and those from vitamin D, which fixates calcium to the bone, which are used for treating postmenopausal osteoporosis.

## Description

### TECHNICAL FIELD OF THE INVENTION

The present invention is directed to compositions that contain risedronate and vitamin D as active ingredients, which are employed as doses that include a safe and efficient quantity of a pharmaceutical composition that includes risedronate and vitamin D as active ingredients and pharmaceutically acceptable excipients. Such dose combines the pharmaceutical properties of risedronate, which prevents bone degradation, and of vitamin D, which fixates calcium to the bone.

### BACKGROUND OF THE INVENTION

The poliphosphoric acids and their pharmaceutically acceptable salt have been used for the treatment and prophylaxis of a number of pathological entities that affect human beings and other mammals, circumstances in which calcium and phosphorus metabolism are involved. These pathological entities can be divided into two large categories:
1. Pathologies characterized by the anomalous mobilization of calcium and phosphorous, a circumstance that determines the general or specific loss of bone (osteoporosis).
2. Pathologies where the levels of calcium and phosphate are extremely high, which generates an abnormal accumulation of these compositions in the body tissues (pathological ossification).

In osteoporosis, there is a disproportionate loss of tissue from the bone and a decrease in the development rate of new bone tissue. This situation results in a loss of bone mass and deterioration of the bone micro architecture (less dense, more fragile, and easy to fracture bone). Osteoporosis can appear at any moment in life but it is more frequent after about age 40, in both men and women. Osteoporosis is classified according to the age when it appears (infantile, postmenopausal and senile), according to the triggering factors (secondary osteoporosis related to the use of steroids), concomitant with another pathologies (rheumatoid arthritis, chronic renal insufficiency, bone metastasis, etc.) Osteoporosis may be a primary bone affection as in Paget's disease, endocrine disease as hyperthyroidism and malignant hyperthermia.

Paget's disease is a metabolic bone disease, characterized by bone destruction and anomalous regeneration resulting in bone deformities.

Osteoporosis clinical manifestations and consequences are similar, regardless of its origin.

Particular bisphosphonates like ethane- 1 -hydroxy- 1, 1 -bisphosphonic acid (EHDP), 3 - amino- 1 -hydroxypropane- 1, 1 -diyl bisphosphonic acid (PDA) and diclorometane bisphosphonic acid (C12, MPD) have been the subject of considerable research efforts. Currently, Paget's disease and heterotopic ossification are treated successfully with EHPD. Bisphosphonates tend to inhibit fine bone tissue degradation, which is beneficial to patients suffering from excessive bone loss. However EHDP, PDA and many other bisphosphonates from previous generations tend to inhibit bone mineralization when they are administered at high doses. Risedronate inhibits osteoclastmediated bone resorption, it is a pyridinyl bisphosphonate with affinity for hydroxyapatite crystals in bone, reduces its resorption and abnormal loss of mineral substances.

In preclinical studies risedronate has demonstrated to have an antiosteclastic effect, therefore it is antiresorptive, thus determining bone mass increment and biomechanical resistance based on the dose. Risedronate's mechanism of action has been confirmed in pharmacodynamic and clinical studies by biochemical markers in bone remodeling.

The effectiveness of risedronate treatment has been proven by measuring the decrease on the rate of biochemical markers for bone remodeling, on the first month, reaching a maximum fall at the end of 3 to 6 months.

The decrease on the rate of biochemical markers for bone remodeling is similar with a daily dose or a weekly dose of risedronate.

Even though Vitamin D daily requirements fluctuate between 200 and 800 IU, they may change during the winter or when solar exposure is very low. Vitamin D deficiency is a risk factor to developing osteopenia and fractures. Blood levels of 1.25 dihydroxy-D < 15 ng/ml (37 mM/L) produce progressive abnormal loss of mineral substances, as much as 80 to 90 percent in the worst cases, as well as hypocalcemia, which results in secondary hyperparathyroidism with increased bone reabsorption.

Calcium absorption increases in the presence of vitamin D3 and the parathyroid hormone. Vitamin D3 is metabolized in the organism resulting in 1.25 - dihydroxycholecalciferol, a metabolite needed for actively transporting calcium in the intestine, since excretion takes part through the kidney. Parathyroid hormone stimulates calcium reabsorption at the kidneys.

The following are risk factors for developing postmenopausal osteoporosis: family history of osteoporosis, smoking, sedentary life and premature menopause among others. The most important clinical consequence of osteoporosis is bone fracture. The increase in the risk of fracture is directly related to the risk factors of osteoporosis. Clinical researches have studied risedronate effects over the risk of hip fracture and vertebrae, including women in premature and late menopause, with or without hip fracture history, with control groups given calcium and vitamin D3. The absolute and relative risk of new hip and vertebrae fractures has been measured based on the analysis of time elapsed until the first fracture occurs.

Such studies have shown that the use of risedronate promotes an increase in mineral bone density, at the femoral neck and radious mid diaphysis, in postmenopausal women taking estrogens/risedronate, compared to the group taking only estrogens.

A moderate decrease remodeling rate in bone has been proven, according to what was expected, in bone biopsy samples belonging to postmenopausal women treated for a period of 2 to 3 years with risedronate. The bone tissue formed during the treatment with risedronate presented a laminar structure and normal mineralization. Such data, along with the lower incidence in fractures associated to osteoporosis in vertebrae, in postmenopausal women, indicates the absence of harmful effects in bone quality.

Treatment with risedronate has demonstrated reduction of the annual height loss in control groups.

The efficacy of bisphosphonates in the treatment of postmenopausal osteoporosis is more significant in patients with low bone mineral density (bone mineral density T score of L -2.5 standard deviations in the hip or femoral neck) and/or fracture history.

There is limited evidence to prove the efficacy of bisphosphonates in women of advanced age (over 80 years old), due to non-skeletal risk factors more frequent in this age group (for example, bad sight, equilibrium disorder and neurological disease) which are predisposing to falls, and therefore fractures.

Results from clinical research of patients with Paget's disease show that a daily dose of 35mg risedronate for two months:
- Normalized serum alkaline phosphatase concentration in 77 percent of patients, in comparison to 11 percent in the control group (etidronate 400 mg/day for 6 months).
- Significantly reduced the hydroxyproline/urinary creatinine and deoxypyridinoline/urinary creatinine ratio.
- Reduced the dimension of lithic lesions in the axial and appendicular skeleton, as shown in the x-rays taken before and after 6 months of treatment; furthermore, there were no new fractures.

The response observed was similar, regardless if the patient had been treated or not, and the severity of the illness.

Fifty-three percent of patients stayed on biochemical remission during the 18 months following the two-month treatment.

Risedronate absorption is relatively quick after a single oral administration (Tmax. approximately 1 hour) and the speed does not depend on the dose used in the studied range (2.5 mg to 30 mg single dose; 2.5 mg to 5 mg multiple doses daily, until 50 mg weekly). The tablet's mid oral bioavailability is 0.63 percent and reduces when sodium risedronate is administered with food. Bioavailability was similar in men and women.

The average distribution volume in a state of balance is 6.3 l/kg in humans.

There is no evidence of systemic metabolism for sodium risedronate.

Vitamin D3 is absorbed in the intestine and then transformed into 25(OH)D in the liver by microsomal enzymes. It then goes to the kidney where, through 1-alpha hydroxylation becomes 1 -alpha- 25 -dihydroxy-D3. This last composition has a regulating function to increase calcium absorption.

Sodium risedronate is administered in tablets to be absorbed through the small intestine, the same as vitamin D3, and it is prescribed as treatment for osteoporosis in postmenopausal women, osteoporosis prevention in high risk postmenopausal women, prevention and treatment of osteoporosis induced by systemic corticosteroids, in women and men who undergo a systemic treatment with corticosteroids (7.5 mg daily dose or its equivalence in prednisone for 3 months). These patients must receive enough calcium and vitamin D. Treatment of Paget's disease.

Therefore, it is desirable to develop innovating oral doses that combine the benefits of risedronate and vitamin D, which make them useful for osteoporosis prevention and treatment (postmenopausal and senile).

In the technique's description there are several compositions of risederonate active ingredient, like those found in the North American patents numbers 5,935,602; 6,096,342 and 5,569,460. However none of them have the advantages of the present invention. On the other hand, publication WO 93/09785 from May 19th 1993, describes a first composition of risedronate that differs significantly from the present invention. The North American patents 5,622,721 and 6,596,701 describe other risedronate compositions, however the components and the manufacturing process are less efficient, less secure and more expensive that the one described in this invention.

### SUMMARY OF THE INVENTION

The present invention describes compositions of risedronate and vitamin D3, which can be manufactured in oral doses with effective amounts of risedronate and vitamin D3 to be delivered to the small intestine, prevent bone degradation and fixate calcium. They are useful in the treatment of postmenopausal osteoporosis (it is possible to leave senile and postmenopausal osteoporosis), with a significant reduction in the risk of bone fractures.

### INVENTION ESPECIFICATIONS

This invention focuses on compositions of risedronate and vitamin D3 manufactured in oral doses that include safe and effective quantities of an active ingredient of both substances, and pharmaceutically acceptable excipients.

The term "risedronate", as used in this document, refers to 3-pyridil-1-hydroxyethylyden-1,1-bisphosphonic acid.

Risedronate as a composition is described with more detail in the publications attached as reference: application form for patent "EPO 0.186,405 from Benedict et al., assigned to Procter&Gamble Co.", published on July 2° 1986.

The term "Vitamin D3" and/or "Vitamin D", as used in this document, refers to (3β,5Z,7E)-9,10-secocholesta-5,7,10(19)-trien-3-ol, IUPAC (International Union of Pure and Applied Chemistry) nomenclature C27H44O, PM 384.64, which has the generic name cholecalciferol.

The term "risedronate active ingredient" includes risedronate, risedronate salts and esters or any mixture of them. Any pharmaceutically acceptable, non-toxic salt or ester from risedronate may be used as active ingredient for the innovating forms of oral doses in this invention. Risedronate salts may be acid adding, particularly chlorohydrate, also pharmaceutically acceptable, non-toxic organic or inorganic acid salts may be used. Likewise, alkaline salts, calcium and magnesium, but preferably calcium and sodium. Particularly, other risedronate esters which are convenient as active ingredients in the present invention are the straight or ramified chain alkylic C1-C18 esters, including, but not limited to methyl, ethyl, propyl, isopropyl, butyl , isobutyl, amylic, hexil, heptil, octal, nonil, decyl, lauryl, myristyl, cetilyc and estearyl; straight and branched alkenyl C1-C18 esters, including, but not limited to vynil, alkyl, undecenyl and lynoleyl; cycloalkyl esters C3-C8, including, but not limited to cyclopropyl, cyclobutyl, cyclopentyl, cyclohexyl, cycloheptyl and cyclooctyl; aryl esters, including, but not limited to phenyl, toluyl, xylyl and naphtyl; alicyclic esters, including, but not limited to mentyl; aralkyl esters, including, not limited to benzyl and fenetyl. Generally speaking, the appropriate active ingredient selection of risedronate depends on the type of formulation, the disease, particularly the location and type of disease, and the amount of active ingredient desired to be released. Also, the active ingredient's physical and chemical characteristics must be considered to choose the pharmaceutically acceptable and convenient excipient for the dose including risedronate active ingredient.

Pharmaceutical acceptable excipients include polymers, resins and plasticizers, filling agents, lubricants, solvents, surfactants, preservers, sweeteners, flavoring, buffers, colorants, pharmaceutically degree pigments and viscosity agents.

Notwithstanding the above, the present invention demonstrates that it is only necessary to use filling agent such as microcrystalline cellulose PH200 or lactose: agglutination agents such as povidone and crospovidone: magnesium stearate as lubricant and disintegrating agents such as croscarmellose sodium, to obtain a composition with better dissolution characteristics.

The covering known for the present invention is known in the state of the technique, and those containing L30D55 and Eudracolor™, preferably Blue 026, are preferred.

The efficient dose of the active ingredient of risedronate and vitamin D3 depends on the type and stage of the disease, and for adults it generally reaches approximately 25 mg to 40 mg daily, preferably from 30 mg to 35 mg daily. When the drug must be administered continuously, the preferred dose is 30 mg to 35 mg daily, preferably 35 mg a day.

Even though vitamin D3 daily dietary requirements fluctuate from 200 IU to 800 IU, and they may be modified during winter, or when solar exposition is very low. However, for the cases mentioned in this document, a dose 2,500 IU to 3,000 IU is recommended, preferably 2,800 IU.

A human being or other mammal suffering from calcium and phosphate metabolism disease may be successfully treated by delivering risedronate and vitamin D3 active ingredient to the small intestine.

The pharmaceutical compositions described below include from 15 to 20 percent of risedronate active ingredient, preferably 17 to 18 percent; from 0.03 to 0.04 percent of vitamin D3 active ingredient and from 75 to 85 percent pharmaceutically acceptable excipient.

The term "oral dose form" as used in these document stands for any pharmaceutical composition thought to be administered orally to an individual's gastrointestinal tube.

Risedronate and vitamin D3 coated tablets are made out of coating and tablets risedronate and vitamin D3 active ingredient.

The process to manufacture risedronate and vitamin D3 is described as follow:

The composition that contains active ingredients is prepared with the next excipients:

Croscarmellose sodium and vitamin D3 are previously strained through a No. 20 screen, and mixed for 15 minutes with a "V" mixer. On a different No. 20 screen, microcrystalline cellulose PH 200, risedronate and Ludipress™ are also strained. Fifty percent of microcrystalline cellulose PH 200 and 30 percent of Ludipress™ are added into the "V" mixer and mixed for 30 minutes, after that, the other 50 percent of microcrystalline cellulose PH 200 and another 30 percent Ludipress™ are added and mixed for another 30 minutes. With the mixture still in the "V" mixer, risedronate is added and mixed for 45 minutes. The other 40 percent of Ludipress™ is added and mixed for 5 minutes. Magnesium stearate, previously strained through a No. 30 screen, is added and mixed with the rest of the ingredients for 5 minutes. The tablets are made using tablet press machine, and then they are coated with Eudragit™ L30D55 and Eudracolor™ Blue 026, according to the method already known in the state of the technique, thus obtaining a 10.5 percent increase in the weight of the nucleus.

## Claims

1. Innovative oral dose that includes 15 to 20 percent of risedronate and vitamin D3 active ingredient, preferably 17 to 18 percent of risedronate active ingredient and 0.03 to 0.04percent of vitamin D3 active ingredient and 75 to 85 percent of pharmaceutically acceptable excipient.

2. Innovative oral dose that includes risedronate and vitamin D3 active ingredient; namely, approximately 20 mg to 40 mg of risedronate, preferably from 30 mg to 35 mg daily; 0.03 mg to 0.04 mg of vitamin D3, preferably 0.07mg, when the dose has to be administered continuously, and 140 mg to 180 mg of pharmaceutical excipients.

3. Innovative oral dose of risedronate and vitamin D3 active ingredient according to any of the previous claims, where such dose is administered daily for at least 2 months.

4. A dose according to any of the previous claims where risedronate active component is selected from the group consisting of risedronate salts and esters or any mixture of any of them; risedronate salts may be acid adding salts, particularly chlorohydrate, also pharmaceutically acceptable, non-toxic organic or inorganic acid salts; alkaline salts (calcium and magnesium), sodium salt. Other risedronate esters are the straight or ramified chain alkylic C1-C18, including, but not limited to methyl, ethyl, propyl, isopropyl, butyl , isobutyl, amylic, hexil, heptil, octal, nonil, decyl, lauryl, myristyl, cetilyc and estearyl; straight and branched alkenyl C2-C8 esters, including, but not limited to vynil, alkyl, undecenyl and lynoleyl; cycloalkyl esters C3-C8, including, but not limited to cyclopropyl, cyclobutyl, cyclopentyl, ciclohexyl, cicloheptyl, ciclooctyl; aryl esters, including, but not limited to phenyl, toluyl, xylyl and naphthyl; alycyclic esters, including, but not limited to metyl; aralkyl esters, including, not limited to benzyl and fenetyl.

5. A dose according to the previous claims where the active ingredient is sodium risedronate.

6. A dose according to the previous claims where the pharmaceutically acceptable excipients include de filling agents, viscosity agents, sweeteners and lubricants.

7. A dose according to the previous claims where the filling and agglutination agent is microcrystal cellulose PH 200, the filling agents are crospovidone, povidone and lactose; the lubricant agent is magnesium stearate and the disintegrating agent is sodium croscarmellose.

8. A dose according to the previous claims where the filling agent is 93percent lactose, 3.5 percent crospovidone and 3.5 percent povidone.

9. The use of the composition of claim 1, for preparing a single unit drug, where the drug is used to treat and/or prevent postmenopausal osteoporosis.

10. The process to produce a dose according to claim 1 is as follows:
- Strain sodium croscarmellose and vitamin D3 with No. 20 screen,
- Mix the previous components in a "V" mixer for 15 minutes,
- Strain microcrystal cellulose PH 200, risedronate and Ludipress™ with No.20 screen,
- Add 50 percent of microcrystal cellulose PH 200 and 30 percent of Ludipress™ to the "V" mixer and mix for 30 minutes,
- Add the other 50 percent microcrystal cellulose PH 200 and 30 percent more of Ludipress™ and mix for 30 minutes,
- Without removing the mixture from the "V" mixer add risedronate and mix for 45 minutes,
- Add the other 40 percent of Ludipress™ and mix for 5 minutes,
- Add magnesium stearate, previously strained with No. 30 screen, and mix with the rest of the ingredients for 5 minutes,
- Tablets are made using tablet press machine.
- Coat with Eudragit™ L30D55 and Eudracolor™ Blue 026, according to the method already known in the state of the technique, obtaining a 10 to 11 percent increase in the weight of the nucleus.
